# FASCICULE DE BREVET EUROPEEN

(11) **EP 0 813 845 B1**
(45) Date de publication et mention de la délivrance du brevet: **30.10.2002**
(21) Numéro de dépôt: 97401435.9
(22) Date de dépôt: 20.06.1997
(51) Int. Cl.: A61B 17/70

(54) **Dispositif de liaison transversale de tiges de support d'implants rachidiens**
Querverbindung für Stützstäbe für Wirbelsäulenimplantate
Transverse connector for spinal implant support rods

(30) Priorité: 20.06.1996 FR 9607671
(43) Date de publication de la demande: 29.12.1997
(73) Titulaire: Euros, 13600 La Ciotat (FR)
(72) Inventeur: Mercier, Pierre, Saint-Augustin De Demaures, Quebec (CA)
(74) Mandataire: CABINET BONNET-THIRION

(56) Documents cités:
- EP-A- 0 676 177
- DE-A- 4 447 002
- US-A- 5 330 473

## Description

La présente invention concerne un dispositif de liaison transversale de deux tiges de support d'implants rachidiens.

Plus particulièrement, elle concerne un dispositif comprenant au moins un élément de serrage apte à enserrer une tige de support, un élément de liaison transversale s'étendant entre les deux tiges de support ainsi qu'un système de verrouillage apte à maintenir assemblés ladite tige de support, ledit élément de serrage et ledit élément de liaison transversale.

Un tel dispositif permet de lier ensemble des tiges moletées d'un dispositif destiné à assurer le maintien du rachis selon une courbure appropriée. Ce dispositif de maintien est utilisé notamment dans le traitement des arthroses, des fractures vertébrales ou bien pour corriger des déviations de la colonne vertébrale telles q'une scoliose ou une cyphose.

On connaît déjà un dispositif de liaison transversale dans lequel l'élément de serrage comporte un système de mâchoires ouvert vers le bas apte à enserrer une première tige de support, ces mâchoires étant perçées d'un alésage traversant pour la mise en place de l'élément de liaison transversale selon une direction transversale à la direction de la première tige support, dans un plan parallèle à celle-ci, et un système de verrouillage d'une tige de support et de l'élément de liaison transversale dans lesdites mâchoires. Le système de verrouillage de ce dispositif connu est constitué par deux écrous vissés de part et d'autre desdites mâchoires sur l'élément de liaison transversale constitué par une tige introduite transversalement dans lesdites mâchoires. Afin de pouvoir régler l'écartement des deux éléments de serrage l'un par rapport à l'autre sur la tige de liaison transversale, cette dernière comprend à une extrémité, une molette qui permet de visser la tige de liaison transversale dans des écrous situés de part et d'autre des mâchoires d'un élément de serrage, maintenue bloquée. Une fois que l'écartement desdits éléments de serrage l'un par rapport à l'autre est déterminé, le chirurgien coupe la tige de liaison transversale au niveau de l'écrou situé du côté de la molette afin de désolidariser la molette de la tige de liaison transverale.

La mise en place et le serrage des éléments de serrage sur les tiges moletées, positionnées de part et d'autre d'une vertèbre, et liées à des implants rachidiens fixés aux vertèbres s'avèrent généralement une étape longue et difficile pour le chirurgien, cette étape intervenant généralement à la fin de l'intervention sur le patient. En effet, le serrage des éléments de serrage sur les tiges moletées nécessite le vissage des écrous sur la tige de liaison transversale afin de fermer les mâchoires de chaque élément de serrage sur la tige moletée correspondante. Pour chaque couple de mâchoires, le chirurgien doit intervenir avec une première clé positionnée sur un écrou pour le maintenir fixe par rapport à la tige de liaison transversale et une deuxième clé de façon à visser l'écrou correspondant, et réciproquement, sur la tige de liaison et rapprocher ainsi les mâchoires qui viennent pincer lesdites tiges moletées. Ces écrous se situés sur les côtés latéraux de la mâchoire de part et d'autre de celle-ci et sont difficiles d'accés sachant que les mâchoires sont entourées de chair. DE-A-4 447 002 divulgue un dispositif de liaison transversale montrant les caractéristiques du préambule de la revendication 1.

Par ailleurs, on connaît un dispositif de liaison transversale du type de l'invention qui fait l'objet du brevet français FR-A-2 732 887 appartenant à la demanderesse.

Plus particulièrement, ce dispositif de liaison transversale comprend un élément de serrage pourvu d'un corps muni d'un système de mâchoires ouvert vers le bas, apte à enserrer une tige de support, ledit corps étant percé d'un alésage traversant pour la mise en place d'une tige de liaison transversale selon une direction transversale dans un plan parallèle par rapport à la tige de support, et un système de verrouillage de la tige de support et de la tige de liaison transversale dans ledit corps. Le système de mâchoires de l'élément de serrage est formé par une partie fixe du corps et une partie mobile entre une position d'engagement de la tige de support dans le système de mâchoires et une position de serrage de la tige de support entre les parties fixe et mobile dudit système de mâchoires. Le système de verrouillage de ce dispositif comporte un organe de serrage fileté qui traverse le corps, cet organe de serrage fileté étant lié à la partie mobile du système de mâchoires, est vissé dans un écrou positionné sur le dessus du corps du fait que le vissage de l'écrou sur le corps entraîne le déplacement de la partie mobile entre la position d'engagement et la position de serrage.

Un tel dispositif est mis en place rapidement sur les tiges moletées placées de part et d'autre d'une vertèbre et présente l'avantage que pour chaque élément de serrage l'écrou est positionné sur le dessus du corps dudit élément et fait face au chirurgien qui peut de ce fait l'atteindre facilement. Ainsi, en une seule opération de vissage de l'écrou sur le corps il verrouille alors l'élément de serrage sur ladite tige moletée et sur la tige de liaison transversale.

Toutefois, ce dispositif précité présente comme principale inconvénient le fait que l'élément de serrage a une forme complexe avec une partie mobile liée au système de verrouillage.

Le but de la présente invention est alors de proposer un nouveau dispositif de liaison transversale dans lequel l'élément de serrage est de conception simple pouvant alors être mis en place rapidement sur une tige de support d'implants rachidiens et dans lequel l'élément de serrage et le système de verrouillage sont agencés de sorte qu'ils permettent un verrouillage rapide dudit élément de serrage sur ladite tige de support.

Plus particulièrement, selon l'invention, chaque élément de serrage est réalisé en un matériau élastiquement déformable et comprend une paroi inférieure pourvue d'un évidement en forme de U ouvert vers le bas destiné à être engagé sur la tige de support ainsi qu'une paroi supérieure de forme sensiblement concave, l'élément de liaison transversale comprenant au moins une portion d'extrémité plate apte à s'appliquer sur ladite paroi supérieure sensiblement concave de chaque élément de serrage, le système de verrouillage comportant d'une part une tige de verrouillage apte à être positionnée en saillie vers le haut de ladite paroi supérieure dudit élément de serrage de manière à traverser ledit élément de liaison transversale et d'autre part un organe de verrouillage apte à coopérer avec la tige de verrouillage pour être serré contre l'élément de liaison transversale et provoquer le serrage de la portion d'extrémité plate dudit élément de liaison transversale contre ladite paroi supérieure sensiblement concave dudit élément de serrage et par là même, le rapprochement mutuel des parois latérales de l'évidement en forme de U dudit élément de serrage contre ladite tige de support par déformation élastique dudit élément de serrage.

Ainsi, par une seule action de serrage de l'organe de verrouillage sur la tige de verrouillage contre l'élément de liaison transversale, il est possible de bloquer l'élément de serrage sur la tige de support par déformation élastique de celui-ci. On a alors un serrage rapide du dispositif de liaison transversale sur les tiges de support d'implants rachidiens ce qui permet de raccourcir considérablement le temps de l'intervention chirurgicale.

Bien entendu, l'élément de serrage du dispositif conforme à l'invention présente une forme judicieuse de sorte que sa déformation en cours du serrage reste élastique. Cela permet un éventuel démontage du dispositif.

Un tel dispositif est simplifié par rapport au dispositif du brevet FR-A-2 732 887 appartenant à la demanderesse, et de ce fait sa mise en place est encore plus rapide.

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique en perspective d'un mode de réalisation du dispositif de liaison transversale conforme à l'invention à l'état monté sur deux tiges de support d'implants rachidiens,
- la figure 2 est une vue schématique éclatée du dispositif de liaison transversale de la figure 1,
- la figure 3 est une vue schématique en perspective d'un élément de serrage d'une variante de réalisation du dispositif de liaison transversale conforme à l'invention.

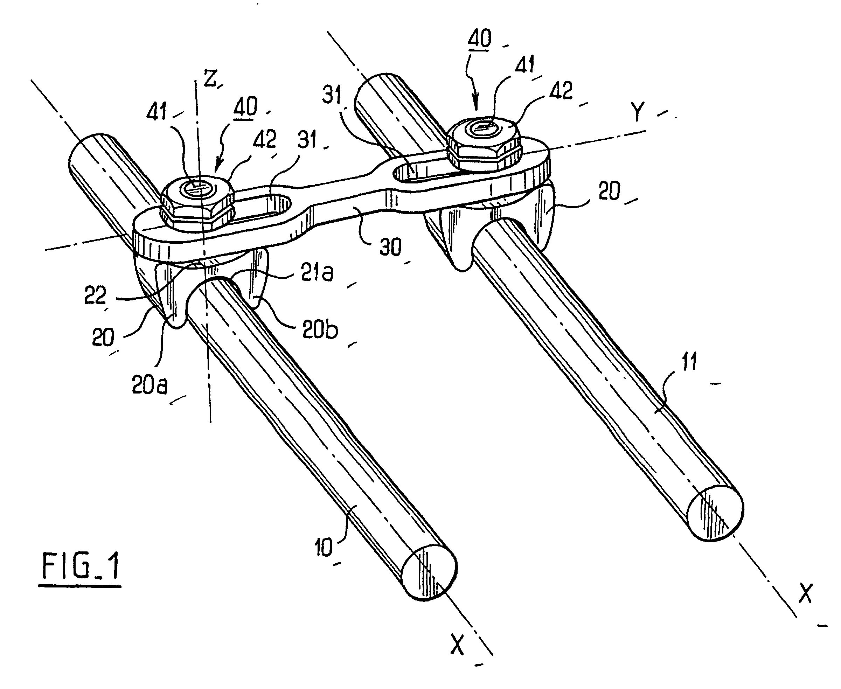

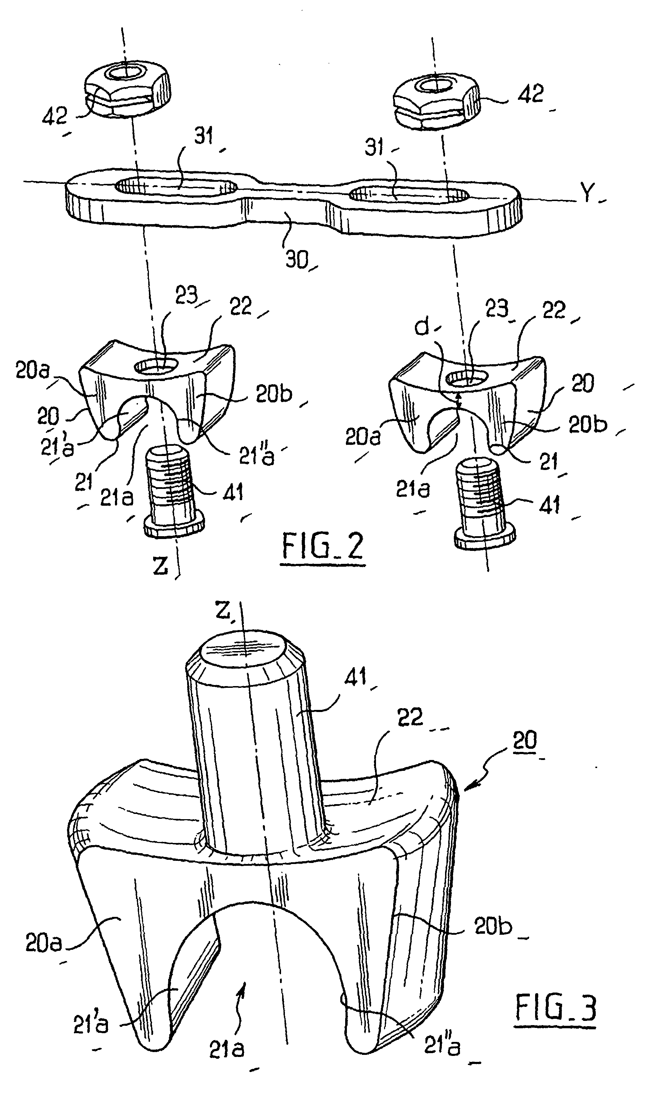

Sur les figures 1 et 2, on a représenté un dispositif de liaison transversale de deux tiges support 10, 11 d'implants rachidiens (ici non représentés) qui font partie d'un dispositif de maintien du rachis. Ces deux tiges de support 10, 11 sont des tiges moletées positionnées de part et d'autre des vertèbres ici de manière sensiblement parallèles selon l'axe X.

En fonction de l'intervention chirurgicale, il peut s'avèrer que ces tiges de support 10, 11 soient légèrement cintrées et positionnées de manière à former un angle.

Le dispositif de liaison transversale représenté sur ces figures comporte deux éléments de serrage 20. Chaque élément de serrage 20 est apte à enserrer une tige de support 10, 11. Le dispositif comporte en outre un élément de liaison transversale 30 s'étendant selon l'axe transversal Y, entre les tiges de support 10, 11, et destiné à être lié à chaque élément de serrage 20, ainsi que pour chaque élément de serrage 20 un système de verrouillage 40 apte à maintenir assemblés une tige support 10, 11, l'élément de serrage correspondant 20 et ledit élément de liaison transversale 30.

Chaque élément de serrage 20 est formé d'une seule pièce réalisée en un matériau élastiquement déformable du type titane ou inox. Il comprend une paroi inférieure 21 pourvue d'un évidement 21a en forme de U ouvert vers le bas, destiné à être engagé sur une tige support 10, 11, et une paroi supérieure 22 de forme sensiblement concave, c'est-à-dire en creux.

On entend ici par vers le bas en direction de la chair du patient.

Comme on peut le voir sur les figures, la paroi supérieure 22 et la paroi inférieure 21 de l'élément de serrage 20 définissent entre elles deux mâchoires 20a, 20b aptes à être positionnées de part et d'autre de la tige de support 10, 11 correspondante.

En outre, l'élément de liaison transversale du dispositif de liaison conforme à l'invention se présente ici sous la forme d'une barrette 30 s'étendant selon l'axe Y entre les tiges de support 10, 11. Cette barrette 30 comporte des portions d'extrémité plates élargies aptes à s'appliquer sur les parois supérieures 22 sensiblement concaves desdits éléments de serrage 20. Chaque portion d'extrémité plate de ladite barrette 30 comprend un évidement allongé 31 de forme oblongue s'étendant selon l'axe Y.

Le système de verrouillage 40 du dispositif de liaison transversale plus particulièrement représenté sur les figures 1 et 2, comporte pour chaque élément de serrage 20 une tige de verrouillage 41, ici une tige filetée séparée, destinée à s'engager verticalement selon un axe Z dans un alésage traversant vertical 23 prévu dans chaque élément de serrage 20.

Cet alésage traversant vertical 23 débouche d'une part sur la paroi supérieure 22 de l'élément de serrage et d'autre part dans le fond de l'évidement 21a en forme de U prévu dans la paroi inférieure 21 de celui-ci. Il a pour axe, l'axe vertical Z.

La tige de verrouillage filetée 41 du système de verrouillage 40 est engagée dans l'alésage 23 du côté de l'évidement inférieur 21a dudit élément de serrage 20 de sorte que la tête de la tige filetée 41 se positionne dans le fond de l'évidement 21a et le corps de ladite tige fait saillie vers le haut de ladite paroi supérieure 22 dudit élément de serrage 20. Comme on peut le voir sur la figure 1, chaque tige filetée de verrouillage 41 en position dans un élément de serrage 20 traverse l'évidement allongé de forme oblongue 31 prévu dans une portion d'extrémité plate de la barrette 30 appliquée contre la paroi supérieure 22 de l'élément de serrage 20.

En outre, le système de verrouillage 40 comporte pour chaque tige de verrouillage filetée 41, un écrou 42 constituant un organe de verrouillage destiné à être vissé sur la tige filetée 41 pour être serré contre une portion d'extrémité plate de la barrette 30.

Le verrouillage d'un élément de serrage sur une tige moletée de support d'implants rachidiens s'effectue de la façon suivante.

Lorsque l'écrou 42 est vissé sur la tige filetée 41 positionnée sur l'élément de serrage 20 et traversant la barrette 30, ledit écrou 42 vient prendre appui sur ladite barrette 30 et par là même exerce une pression sur ladite portion d'extrémité plate de la barrette 30 qui prend appui contre la surface supérieure 22 sensiblement concave de l'élément de serrage 20. Ainsi, la portion d'extrémité plate de la barrette 30 exerce une pression sur la surface supérieure sensiblement concave 22 de l'élément de serrage 20 en la déformant élastiquement pour l'applanir. Cette déformation provoque le rapprochement mutuel des parois latérales 21'a, 21"a de l'évidement inférieur 21a dudit élément de serrage 20 contre la tige de support correspondante 10, 11.

On a alors une déformation élastique de l'élément de serrage 20 qui vient se refermer comme une pince sur la tige de support 10, 11.

La concavité de la surface supérieure 22 de l'élément de serrage 20, la profondeur de l'évidement 21a en forme de U dudit élément de serrage 20 et la distance d entre le fond de la surface supérieure 22 et le fond de l'évidement 21a sont calculées de sorte que lorsque l'écrou de verrouillage est complétement vissé sur la tige de verrouillage la déformation élastique de la paroi supérieure de l'élément de serrage entraine un serrage optimum de la tige de support.

On notera que l'élément de serrage 20 est dimensionné de façon telle que sa déformation reste élastique lors de son serrage sur la tige de support 10. Ceci permet un démontage éventuel par dévissage de l'écrou 42 sur la tige 41.

Selon une variante de réalisation représentée sur la figure 3, on remarquera que l'élément de serrage 20 et la tige de verrouillage 41 forment une seule pièce monobloc.

On notera que les tiges de verrouillage peuvent coulisser ou encore pivoter légèrement dans les évidements allongés de forme oblongue de la barrette 30.

On peut alors par coulissement et/ou pivotement dans lesdits évidements oblongs desdites tiges le verrouillage 41 solidarisé des éléments de serrage 20 rapprocher mutuellement lesdits éléments de serrage 20 et les orienter l'un par rapport à l'autre en fonction de la position relative des tiges de support 10, 11.

Le pivotement et la translation autorisés de cet ensemble élément de serrage 20, tige de verrouillage 42, et écrou 41 permettent de lier deux tiges de support d'implants rachidiens qui ne sont pas positionnées exactement parallèles l'une à l'autre mais qui peuvent présenter entre elles des distances variables.

## Revendications

1. Dispositif de liaison transversale de deux tiges de support (10, 11) d'implants rachidiens, comprenant au moins un élément de serrage (20) apte à enserrer une tige de support (10), un élément de liaison transversale (30) s'étendant entre les deux tiges de support (10, 11) ainsi qu'un système de verrouillage (40) apte à maintenir assemblés ladite tige de support (10), ledit élément de serrage (20) et ledit élément de liaison transversale (30), **caractérisé en ce que** chaque élément de serrage (20) est réalisé en un matériau élastiquement déformable et comprend une paroi inférieure (21) pourvue d'un évidement (21a) en forme de U ouvert vers le bas destiné à être engagé sur la tige de support (10) ainsi qu'une paroi supérieure (22) de forme sensiblement concave, **en ce que** l'élément de liaison transversale (30) comprend au moins une portion d'extrémité plate apte à s'appliquer sur ladite paroi supérieure (22) sensiblement concave de chaque élément de serrage (20), et **en ce que** le système de verrouillage (40) comporte d'une part une tige de verrouillage (41) apte à être positionnée en saillie vers le haut de ladite paroi supérieure (22) dudit l'élément de serrage (20) de manière à traverser ledit élément de liaison transversale (30) et d'autre part un organe de verrouillage (42) apte à coopérer avec la tige de verrouillage (41) pour être serré contre l'élément de liaison transversale (30) et provoquer le serrage de la portion d'extrémité plate dudit élément de liaison transversale (30) contre ladite paroi supérieure (22) sensiblement concave dudit l'élément de serrage (20) et par là même le rapprochement mutuel des parois latérales de l'évidement (21a) en forme de U dudit élément de serrage (20) contre ladite tige du support (10) par déformation élastique dudit élément de serrage (20).

2. Dispositif de liaison transversale selon la revendication 1, **caractérisé en ce que** chaque portion d'extrémité plate dudit élément de liaison transversale (30) comporte un évidement allongé (31) s'étendant selon un axe Y sensiblement transversal à l'axe X desdites tiges de support (10,11), destiné à être traversé par ladite tige de verrouillage (41) du système de verrouillage (40) solidarisée à l'élément de serrage (20), ladite tige de verrouillage (41) étant apte à coulisser et/ou pivoter dans ledit évidement allongé (31).

3. Dispositif de liaison transversale selon l'une des revendications 1 ou 2, **caractérisé en ce que** la tige de verrouillage (41) du système de verrouillage (40) et ledit élément de serrage (20) forment une seule pièce monobloc.

4. Dispositif de liaison selon l'une des revendications 1 ou 2, **caractérisé en ce que** chaque élément de serrage (20) comporte un alésage traversant vertical (23) destiné à être traversé par ladite tige de verrouillage (41) séparée dudit élément de serrage (20).

5. Dispositif de liaison selon l'une des revendications 1 à 4, **caractérisé en ce que** chaque élément de serrage (20) est réalisé en titane.

6. Dispositif de liaison selon l'une des revendications 1 à 4, **caractérisé en ce que** chaque élément de serrage (20) est réalisé en inox.

7. Dispositif de liaison transversale selon l'une quelconque des revendications 1 à 6, **caractérisé en ce que** le système de verrouillage (40) comprend une tige filetée (41) sur laquelle vient se visser un écrou (42).

8. Dispositif de liaison transversale selon l'une quelconque des revendications 1 à 7, **caractérisé en ce que** l'élément de liaison transversale (30) se présente sous la forme d'une barrette plate comportant des portions d'extrémité élargies.

## Patentansprüche

1. Vorrichtung zur Querverbindung von zwei Stützstäben (10,11) für Wirbelsäulenimplantate, umfassend mindestens ein Klemmelement (20), das einen Stützstab (10) einklemmen kann, ein sich zwischen den beiden Stützstäben (10,11) erstreckendes Querverbindungselement (30) sowie ein Verriegelungssystem (40), das den Stützstab (10), das Klemmelement (20) und das Querverbindungselement (20) zusammengebaut halten kann, **dadurch gekennzeichnet, daß** jedes Klemmelement (20) aus einem elastisch verformbaren Werkstoff hergestellt ist und eine untere Wand (21) aufweist, die mit einer nach unten offenen U-förmigen Aussparung (21a) versehen ist, die dazu bestimmt ist, auf den Stützstab (10) aufgesteckt zu werden, sowie eine obere Wand (22) von im wesentlichen konkaver Form, daß das Querverbindungselement (30) mindestens einen flachen Endbereich aufweist, der sich an die im wesentlichen konkave obere Wand (22) jedes Klemmelements (20) anlegen kann, und daß das Verriegelungssystem (40) einerseits einen Verriegelungsschaft (41) aufweist, der von der oberen Wand (22) dieses Klemmelements (20) nach oben vorstehend so positioniert werden kann, daß er das Querverbindungselement (30) durchquert, und andererseits ein Verriegelungsorgan (42), das mit dem Verriegelungsschaft (41) zusammenwirken kann, um an das Querverbindungselement (30) angepresst zu werden und die Anpressung des flachen Endbereichs des Querverbindungselements (30) an die im wesentlichen konkave obere Wand (22) des Klemmelements (20) und dadurch die gegenseitige Annäherung der Seitenwände (21a) der U-förmigen Aussparung (21a) des Klemmelements (20) gegen den Stützstab (10) durch elastische Verformung des Klemmelements (20) zu bewirken.

2. Querverbindungsvorrichtung nach Anspruch 1, **dadurch gekennzeichnet, daß** jeder flache Endbereich des Querverbindungselements (30) eine langgestreckte Aussparung (31) aufweist, die sich in einer Achse Y erstreckt, die zu der Achse X der Stützstäbe (10,11) quer verläuft, und die dazu bestimmt ist, von dem mit dem Klemmelement (20) fest verbundenen Verriegelungsschaft (41) des Verriegelungssystems (40) durchquert zu werden, wobei der Verriegelungsschaft (41) in der langgestreckten Aussparung (31) gleiten und/oder sich verschwenken kann.

3. Querverbindungsvorrichtung nach einem der Ansprüche 1 oder 2, **dadurch gekennzeichnet, daß** der Verriegelungaschaft (41) des Verriegelungssystems (40) und das Klemmelement (20) ein einziges einstückiges Teil bilden.

4. Verbindungsvorrichtung nach einem der Ansprüche 1 oder 2, **dadurch gekennzeichnet, daß** jedes Klemmelement (20) eine durchgehende vertikale Bohrung (23) aufweist, die dazu bestimmt ist, von dem von dem Verriegelungselement (20) getrennten Verriegelungsschaft (41) durchquert zu werden.

5. Verbindungsvorrichtung nach einem der Ansprüche 1 bis 4, **dadurch gekennzeichnet, daß** jedes Klemmelement (20) aus Titan hergestellt ist.

6. Verbindungsvorrichtung nach einem der Ansprüche 1 bis 4, **dadurch gekennzeichnet, daß** jedes Klemmelement aus Inox hergestellt ist.

7. Querverbindungsvorrichtung nach einem der Ansprüche 1 bis 6, **dadurch gekennzeichnet, daß** das Verriegelungssystem (40) einen Gewindeschaft (41) aufweist, auf den eine Mutter (42) aufgeschraubt wird.

8. Querverbindungsvorrichtung nach einem der Ansprüche 1 bis 7, **dadurch gekennzeichnet, daß** das Querverbindungselement (30) in Form einer flachen Leiste mit verbreiterten Endbereichen vorliegt.

## Claims

1. A device for transversely connecting two spinal implant support rods (10, 11), comprising at least one clamping member (20) adapted to grip a support rod (10), a transverse connecting member (30) extending between the two support rods (10, 11), and a locking system (40) adapted to hold together said support rod (10), said clamping member (20) and said transverse connecting member (30), **characterized in that** each clamping member (20) is made of an elastically deformable material and has a lower wall (21) provided with a U-shaped recess (21a) open at the bottom adapted to be engaged over the support rod (10) and a substantially concave upper wall (22), **in that** the transverse connecting member (30) has at least one flat end portion adapted to be applied to said substantially concave upper wall (22) of each clamping member (20), and **in that** the locking system (40) includes a locking rod (41) adapted to be positioned so that it projects upwardly from said upper wall (22) of said clamping member (20) in such a manner as to pass through said transverse connecting member (30) and a locking member (42) adapted to cooperate with the locking rod (41) so as to be clamped against the transverse connecting member (30) and cause the flat end portion of said transverse connecting member (30) to be clamped against said substantially concave upper wall (22) of said clamping member (20) and thereby movement of lateral walls of the U-shaped recess (21a) of said clamping member (20) toward each other and against said support rod (10) by elastic deformation of said clamping member (20).

2. A transverse connection device according to claim 1, **characterized in that** each flat end portion of said transverse connecting member (30) has an elongate opening (31) extending along an axis Y substantially transverse to the axis X of said support rods (10, 11) and adapted to have said locking rod (41) of the locking system (40) fastened to the clamping member (20) passed through it, said locking rod (41) being adapted to slide and/or pivot in said elongate opening (31).

3. A transverse connecting device according to either claim 1 or claim 2, **characterized in that** the locking rod (41) of the locking system (40) and said clamping member (20) are in one piece.

4. A connecting device according to either claim 1 or claim 2, **characterized in that** each clamping member (20) has a vertical bore (23) through it through which said locking rod (41) can be passed when separated from said clamping member (20).

5. A connecting device according to any of claims 1 to 4, **characterized in that** each clamping member (20) is made of titanium.

6. A connecting device according to any of claims 1 to 4, **characterized in that** each clamping member (20) is made of stainless steel.

7. A transverse connecting device according to any of claims 1 to 6, **characterized in that** the locking system (40) includes a threaded stud (41) onto which a nut (42) is screwed.

8. A transverse connecting device according to any of claims 1 to 7, **characterized in that** the transverse connecting member (30) takes the form of a flat strip with wider end portions.
